# EUROPEAN PATENT APPLICATION

(11) **EP 3 170 395 A1**
(43) Date of publication of application: **24.05.2017**
(21) Application number: 16193161.3
(22) Date of filing: 04.12.2009
(51) Int. Cl.: A01N 43/42, A61K 31/439, A61P 25/30, A61P 25/32

(54) **MORPHINAN DERIVATIVES WITH HIGH ORAL BIOAVAILABILITY**

(30) Priority: 19.03.2009 US 161702 P
(62) Divisional of application: 09842019.3
(71) Applicant: Alkermes Pharma Ireland Limited, Dublin 4 (IE)
(72) Inventor: TURNCLIFF, Ryan, Ashland, MA 01721 (US); DDEAVER, Daniel, Franklin, MA 02038 (US); ARNELLE, Derrick, Arlington, MA 02474 (US)
(74) Representative: Harris, Jennifer Lucy

(57) **Abstract**

The instant application relates to morphinan derivatives of formula I with enhanced oral bioavailability for the treatment of diseases associated with opioid receptor activity or blockade including alcohol and opiate addiction.

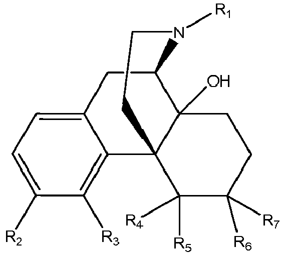

## Description

### RELALTED PARAGRAPH

This claims the benefit of U.S. Provisional Application No. 61/161,702, filed on March 19, 2009. The entire teaching of the above application is incorporated herein by reference.

### TECHNICAL FIELD

This invention relates to mophinan compounds with enhanced oral availability useful as µ, κ and/or δ receptor opioid compounds and pharmaceuticals containing said compounds that may be useful in treating diseases associated with receptor opioid activity or blockade, including but not limited to, mediating analgesia, combating drug and opioid addiction, alcohol addiction, drug overdose, mental illness, bladder dysfunctions, neurogenic bladder, interstitial cystitis, urinary incontinence, premature ejaculation, inflammatory pain, neuropathic pain, cough, lung edema, cardiac disorders, cardioprotection, depression, and cognitive, respiratory, diarrhea, pruritus, irritable bowel syndrome and gastro-intestinal disorders, immunomodulation, and anti-tumor agents.

### BACKGROUND OF THE INVENTION

Opiates have been the subject of intense research since the isolation of morphine in 1805, and thousands of compounds having opiate or opiate-like activity have been identified. Many opioid receptor-interactive compounds including those used for producing analgesia (e.g., morphine) and those used for treating drug addiction (e.g., naltrexone and cyclazocine) have been employed in human therapy. Almost all therapeutically useful opioids in the benzazocine and morphinane classes have a phenolic hydroxyl group (OH) at a position which is numbered "8" in the numbering system used for 2,6-methano-3-benzazocines [e.g., cyclazocine and EKC (ethylketocyclazocine)] and which is numbered "3" in the numbering system used for morphinans (e.g., morphine). When the 3-hydroxyl group is replaced by a number of small, polar, neutral residues, such as carboxamide and thiocarboxamide groups, the adjacent 4-position may be substituted with a hydroxyl to produce compounds with an good affinity for the opioid receptor. Compounds that bind to such receptors are likely to be useful in the treatment of diseases modulated by opiate receptors for example, mediating analgesia, combating drug and opioid addiction, alcohol addiction, drug overdose, mental illness, bladder dysfunctions, neurogenic bladder, interstitial cystitis, urinary incontinence, premature ejaculation, inflammatory pain, peripherally mediated and neuropathic pain, cough, lung edema, diarrhea, pruritis, cardiac disorders, cardioprotection, depression, and cognitive, respiratory, irritable bowel syndrome and gastro-intestinal disorders, immunomodulation, and anti-tumor agents.

Among the therapeutically useful morphinans naltrexone is commonly used for the treatment alcohol and opioid addiction. However, naltrexone is subject to significant first pass metabolism. Furthermore, naltrexone has been found to have significant hepatotoxic effects in high dosages. This has prompted the Food and Drug Administration (FDA) to issue a "black box warning" concerning usage at high doses. As such, there remains a need to develop effective therapeutics for alcohol and opiate addiction which are well tolerated in the body; in particular pharmaceuticals that are effective in low doses and have better pharmacokinetic profile.

### SUMMARY OF THE INVENTION

The present invention relates to the unexpected discovery that certain carboxamide substituted morphinans exhibit enhanced oral bioavailability. The improved bioavailability of carboxamide substituted morphinans provides improved efficacy for the treatment of diseases associated with opioid receptor activity or blockade such as alcohol addiction and opiate dependence.

The present invention relates to the treatment of diseases modulated by opioid receptor activity or blockade by oral administration of compounds of formula I: 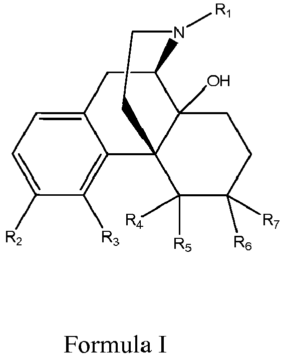 or a pharmaceutically acceptable salt, ester or prodrug thereof, wherein;
R₁ is -(CH₂)ₙ-c-C₃H₅, -(CH₂)ₙ-c-C₄H₇, -(CH₂)ₙ-c-C₅H₉, -(CH₂)ₙ-CH=CH₂ or - (CH₂)ₙ-CH=C(CH₃)₂ wherein n is independently 0, 1, 2 or 3;
R₂ is -CONH₂ or -CSNH₂;
R₃ and R₄ are independently H, -OH or together R₃ and R₄ form an -O- or -S-group;
R₅ is H or C₁₋C₈ alkyl; and
R₆ and R₇ are independently H, -OH, OCH₃ or together R₆ and R₇ form a =O or =CH₂ group.

Compounds of the instant application are useful in the treatment of diseases modulated by opioid receptors activity or blockade, for example, mediating analgesia, combating drug and opiate addiction, alcohol addiction, drug overdose, mental illness, bladder dysfunctions, neurogenic bladder, interstitial cystitis, urinary incontinence, premature ejaculation, inflammatory pain, neuropathic pain, cough, lung edema, diarrhea, pruritus, cardiac disorders, cardioprotection, depression, and cognitive, respiratory, irritable bowel syndrome and gastro-intestinal disorders, immunomodulation, and anti-tumor agents.

### DETAILED DESCRIPTION OF THE INVENTION

Figure 1: Metabolic stability of Compound 1 in cryopreserved hepatocytes.
Figure 2: Concentration-time profile of Compound-1 and naltrexone following IV and PO administration in monkey.
Figure 3: Concentration-time profile of Compound-1 and naltrexone following IV and PO administration in dog.
Figure 4: PK human naltrexone comparison (10 fold decrease in dose).
Figure 5: PK profile of Compound 1 (5 mg) in comparison with naltrexone (50 mg) after oral dosage.

The present invention relates to the use of carboxamide substituted morphinans for the treatment of diseases associated with opioid receptor activity or blockade, in particular opiate and alcohol addiction. The present invention relates to the unexpected discovery that certain carboxamide substituted morphinans exhibit enhanced oral bioavailability. The improved bioavailability of carboxamide substituted morphinans provides improved efficacy for the treatment of diseases associated with opioid receptor activity or blockade such as alcohol addiction and opiate dependence.

Compounds of the instant invention can be obtained by conversion from the phenolic hydroxyl of benzomorphan to a carboxamide moiety. Phenolic hydroxyls of benzomorphan and morphinan derivatives can be chemically converted to carboxamides by a simple, flexible and convenient route described in US patents 6,784,187, 7,262,298 and 7,057,035, and in U.S. Patent Application Publication No. US 2007/0021457 A1, which are all incorporated herein by reference.

In one aspect the invention relates to the treatment of diseases modulated by opioid receptor activity or blockade by oral administration of compounds of formula I: 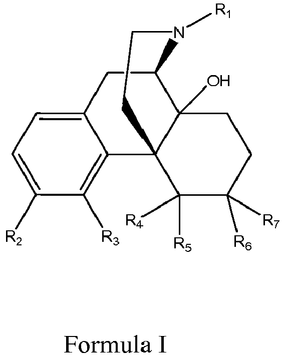 or a pharmaceutically acceptable salt, ester or prodrug thereof, wherein;
R₁ is -(CH₂)ₙ-c-C₃H₅, -(CH₂)ₙ-c-C₄H₇, -(CH₂)ₙ-c-C₅H₉, -(CH₂)ₙ-CH=CH₂ or - (CH₂)ₙ-CH=C(CH₃)₂ wherein n is independently 0, 1, 2 or 3;
R₂ is -CONH₂ or -CSNH₂;
R₃ and R₄ are independently H, -OH or together R₃ and R₄ form an -O- or -S-group;
R₅ is H or C₁₋C₈ alkyl; and
R₆ and R₇ are independently H, -OH, OCH₃ or together R₆ and R₇ form a =O or =CH₂ group.

Representative compounds according Formula I include the following:

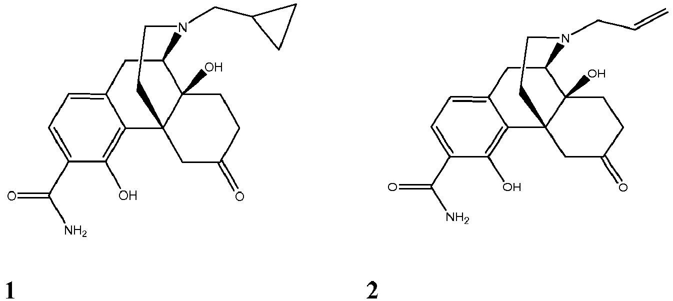

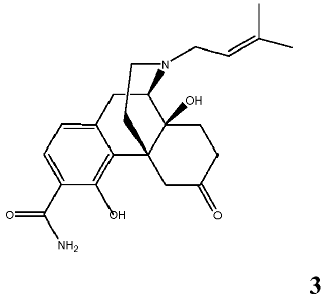

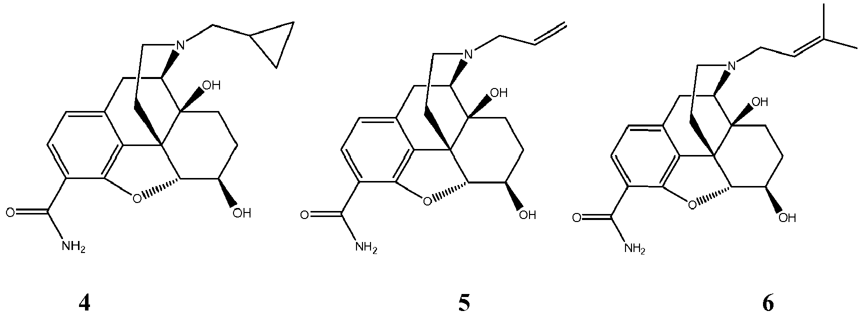

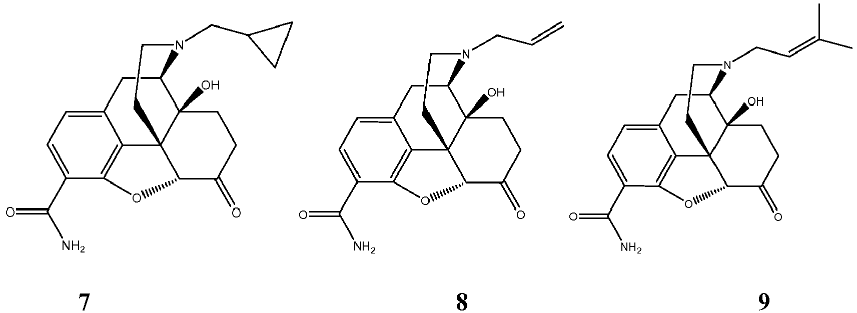

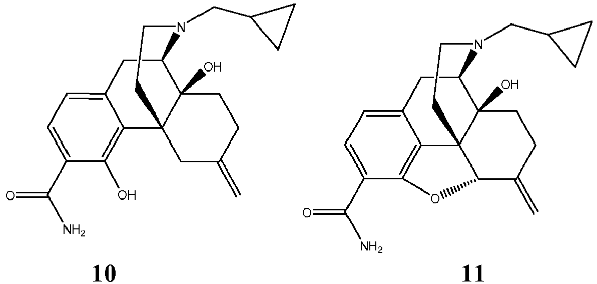

The evaluation of pharmacokinetic (PK) parameters of Compound-1 and naltrexone following oral administration showed a surprisingly better oral bioavailability profile for Compound-1 (Table 1). For monkeys, an improvement in oral bioavailability of over 4 fold was observed. Significant improvement in oral bioavailability was observed in dogs as well as rats. Rats showed an improvement of 4 fold while dogs showed an improvement of over 50 fold. The observed increase in oral bioavailability in comparison with naltrexone was a significant unexpected improvement.

**Table 1: Oral Availability of Compound-1 in comparison with naltrexone.**

| | **Rat** | | **Dog** | | **Monkey** | |
|---|---|---|---|---|---|---|
| | Naltrexone | Compound 1 | Naltrexone* | Compound 1 | Naltrexone | Compound 1 |
| Oral Dose (mg) | 10 | 10 | 1 | 1 | 10 | 10 |
| T_{1/2} (hr) | 2 | 1.2 | 1 | 3.2 | 3 | 5.4 |
| Cₘₐₓ (ng/mL) | 61.6 | 929 | 2.7 | 104 | 13 | 627 |
| AUC_{∞} (ng*hr/mL) | 150 | 1316 | 4.2 | 491 | 54 | 4473 |
| Bioavailability (F%) | 3 | 15 | 1.1 | 66 | 13 | 68 |

| | | | | | | |
|---|---|---|---|---|---|---|
| *Reuning et al. J Pharm Sci 1979 | | | | | | |

The observed increase in bioavailability allows for the administration of lower daily dosages in comparison with naltrexone. For example, where naltrexone is administered in a 50 mg/day dose, Compound-1 is expected to be effective at significantly lower dosage, between about 1.5 to about 20 mg/ day. While the prescribed doses will vary based on clinical evaluation, Compound-1 is expected to be effective in lower doses than naltrexone.

Compounds of the instant application show good to excellent binding affinities to opiate receptors and interfere with the effects of opioid analgesics in the CNS. As such, the compounds of the instant application are useful in the treatment of diseases modulated by opioid receptor activity or blockade, for example, mediating analgesia, combating drug and opioid addiction, alcohol addiction, drug overdose, mental illness, bladder dysfunctions, neurogenic bladder, interstitial cystitis, urinary incontinence, premature ejaculation, inflammatory pain, neuropathic pain, cough, lung edema, diarrhea, pruritus, cardiac disorders, cardioprotection, depression, and cognitive, respiratory, irritable bowel syndrome and gastro-intestinal disorders, immunomodulation, and anti-tumor agents.

### DEFINITIONS

Listed below are definitions of various terms used to describe this invention. These definitions apply to the terms as they are used throughout this specification and claims, unless otherwise limited in specific instances, either individually or as part of a larger group.

The phrase "side effect" refers to a consequence other than the one(s) for which an agent or measure is used, as the adverse effects produced by a drug, especially on a tissue or organ system other than the one sought to be benefited by its administration. In the case, for example, of opioids, the term "side effect" may refer to such conditions as, for example, respiratory depression, acute sedation, constipation, opioid-induced bowel dysfunction, nausea and/or vomiting.

The term "C₁-C₈ alkyl," as used herein, refer to saturated, straight- or branched-chain hydrocarbon radicals containing from one to six, or from one to eight carbon atoms, respectively. Examples of C₁-C₆ alkyl radicals include, but are not limited to, methyl, ethyl, propyl, isopropyl, *n*-butyl, *tert*-butyl, neopentyl, n-hexyl radicals; and examples of C₁-C₈ alkyl radicals include, but are not limited to, methyl, ethyl, propyl, isopropyl, *n*-butyl, *tert-*butyl, neopentyl, n-hexyl, heptyl, octyl radicals.

The compounds described herein contain one or more asymmetric centers and thus give rise to enantiomers, diastereomers, and other stereoisomeric forms that may be defined, in terms of absolute stereochemistry, as (R)- or (S)-, or as (D)- or (L)- for amino acids. The present invention is meant to include all such possible isomers, as well as their racemic and optically pure forms. Optical isomers may be prepared from their respective optically active precursors by the procedures described herein, or by resolving the racemic mixtures. The resolution can be carried out in the presence of a resolving agent, by chromatography or by repeated crystallization or by some combination of these techniques, which are known to those skilled in the art. Further details regarding resolutions can be found in Jacques, et al., Enantiomers, Racemates, and Resolutions (John Wiley & Sons, 1981). When the compounds described herein contain olefinic double bonds or other centers of geometric asymmetry, and unless specified otherwise, it is intended that the compounds include both E and Z geometric isomers. Likewise, all tautomeric forms are also intended to be included. The configuration of any carbon-carbon double bond appearing herein is selected for convenience only and is not intended to designate a particular configuration unless the text so states; thus a carbon-carbon double bond depicted arbitrarily herein as *trans* may be *cis, trans,* or a mixture of the two in any proportion.

The term "subject" as used herein refers to a mammal. A subject therefore refers to, for example, dogs, cats, horses, cows, pigs, guinea pigs, and the like. Preferably the subject is a human. When the subject is a human, the subject may be referred to herein as a patient.

As used herein, and as would be understood by the person of skill in the art, the recitation of "a compound," unless expressly further limited, is intended to include salts, solvates, esters, prodrugs and inclusion complexes of that compound.

As used herein, the term "pharmaceutically acceptable salt" refers to those salts of the compounds formed by the process of the present invention which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art.

Berge, et al. describes pharmaceutically acceptable salts in detail in J. Pharmaceutical Sciences, 66: 1-19 (1977). The salts can be prepared *in situ* during the final isolation and purification of the compounds of the invention, or separately by reacting the free base function with a suitable organic acid. Examples of pharmaceutically acceptable salts include, but are not limited to, nontoxic acid addition salts e.g., salts of an amino group formed with inorganic acids such as hydrochloric acid, hydrobromic acid, phosphoric acid, sulfuric acid and perchloric acid or with organic acids such as acetic acid, maleic acid, tartaric acid, citric acid, succinic acid or malonic acid or by using other methods used in the art such as ion exchange. Other pharmaceutically acceptable salts include, but are not limited to, adipate, alginate, ascorbate, aspartate, benzenesulfonate, benzoate, bisulfate, borate, butyrate, camphorate, camphorsulfonate, carbonate, citrate, cyclopentanepropionate, digluconate, dodecylsulfate, ethanesulfonate, ethanedisulfonate, ethylenediaminetetraacetate (edetate), formate, fumarate, glucoheptonate, glutamate, glycerophosphate, gluconate, hemisulfate, heptanoate, hexanoate, hydroiodide, 2-hydroxyethanesulfonate, hydroxynaphthoate, isethionate, lactobionate, lactate, laurate, lauryl sulfate, malate, maleate, malonate, mandelate, methanesulfonate, mucate, 2-naphthalenesulfonate, nicotinate, nitrate, oleate, oxalate, palmitate, pamoate, pantothenate, pectinate, persulfate, 3-phenylpropionate, phosphate, picrate, pivalate, polygalacturonate, propionate, salicylate, stearate, succinate, sulfate, tannate, tartrate, teoclate, thiocyanate, *p-*toluenesulfonate, undecanoate, valerate salts, and the like. Representative alkali or alkaline earth metal salts include sodium, lithium, potassium, calcium, magnesium, aluminum, zinc and the like. As used herein, the term "pharmaceutically acceptable ester" refers to esters of the compounds formed by the process of the present invention which hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters include, but are not limited to, formates, acetates, propionates, butyrates, acrylates and ethylsuccinates. Further pharmaceutically acceptable salts include, when appropriate, nontoxic ammonium cations and carboxylate, sulfonate and phosphonate anions attached to alkyl having from 1 to 20 carbon atoms.

The term "pharmaceutically acceptable prodrugs" as used herein refers to those prodrugs of the compounds formed by the process of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the present invention. "Prodrug", as used herein means a compound, which is convertible in vivo by metabolic means (e.g. by hydrolysis) to afford any compound delineated by the formulae of the instant invention. Various forms of prodrugs are known in the art, for example, as discussed in Bundgaard, (ed.), Design of Prodrugs, Elsevier (1985); Widder, et al. (ed.), Methods in Enzymology, vol. 4, Academic Press (1985); Krogsgaard-Larsen, et al., (ed). "Design and Application of Prodrugs, Textbook of Drug Design and Development, Chapter 5, 113-191 (1991); Bundgaard, et al., Journal of Drug Deliver Reviews, 8:1-38(1992); Bundgaard, J. of Pharmaceutical Sciences, 77:285 et seq. (1988); Higuchi and Stella (eds.) Prodrugs as Novel Drug Delivery Systems, American Chemical Society (1975); and Bernard Testa & Joachim Mayer, "Hydrolysis In Drug And Prodrug Metabolism: Chemistry, Biochemistry And Enzymology," John Wiley and Sons, Ltd. (2002).

The term "aprotic solvent," as used herein, refers to a solvent that is relatively inert to proton activity, i.e., not acting as a proton-donor. Examples include, but are not limited to, hydrocarbons, such as hexane and toluene, for example, halogenated hydrocarbons, such as, for example, methylene chloride, ethylene chloride, chloroform, and the like, heterocyclic compounds, such as, for example, tetrahydrofuran and N-methylpyrrolidinone, and ethers such as diethyl ether, bis-methoxymethyl ether. Such solvents are well known to those skilled in the art, and individual solvents or mixtures thereof may be preferred for specific compounds and reaction conditions, depending upon such factors as the solubility of reagents, reactivity of reagents and preferred temperature ranges, for example. Further discussions of aprotic solvents may be found in organic chemistry textbooks or in specialized monographs, for example: Organic Solvents Physical Properties and Methods of Purification, 4th ed., edited by John A. Riddick et al., Vol. II, in the Techniques of Chemistry Series, John Wiley & Sons, NY, 1986.

The terms 'protogenic organic solvent" or "protic solvent" as used herein, refer to a solvent that tends to provide protons, such as an alcohol, for example, methanol, ethanol, propanol, isopropanol, butanol, t-butanol, and the like. Such solvents are well known to those skilled in the art, and individual solvents or mixtures thereof may be preferred for specific compounds and reaction conditions, depending upon such factors as the solubility of reagents, reactivity of reagents and preferred temperature ranges, for example. Further discussions of protogenic solvents may be found in organic chemistry textbooks or in specialized monographs, for example: Organic Solvents Physical Properties and Methods of Purification, 4th ed., edited by John A. Riddick et al., Vol. II, in the Techniques of Chemistry Series, John Wiley & Sons, NY, 1986.

Combinations of substituents and variables envisioned by this invention are only those that result in the formation of stable compounds. The term "stable," as used herein, refers to compounds which possess stability sufficient to allow manufacture and which maintains the integrity of the compound for a sufficient period of time to be useful for the purposes detailed herein (e.g., therapeutic or prophylactic administration to a subject).

The synthesized compounds can be separated from a reaction mixture and further purified by a method such as column chromatography, high pressure liquid chromatography, or recrystallization. Additionally, the various synthetic steps may be performed in an alternate sequence or order to give the desired compounds. In addition, the solvents, temperatures, reaction durations, etc. delineated herein are for purposes of illustration only and variation of the reaction conditions can produce the desired bridged macrocyclic products of the present invention. Synthetic chemistry transformations and protecting group methodologies (protection and deprotection) useful in synthesizing the compounds described herein include, for example, those described in R. Larock, Comprehensive Organic Transformations, VCH Publishers (1989); T.W. Greene and P.G.M. Wuts, Protective Groups in Organic Synthesis, 2d. Ed., John Wiley and Sons (1991); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995).

The compounds of this invention may be modified by appending various functionalities via synthetic means delineated herein to enhance selective biological properties. Such modifications include those which increase biological penetration into a given biological system (e.g., blood, lymphatic system, central nervous system), increase oral availability, increase solubility to allow administration by injection, alter metabolism and alter rate of excretion.

### PHARMACEUTICAL COMPOSITIONS

The pharmaceutical compositions of the present invention comprise a therapeutically effective amount of a compound of the present invention formulated together with one or more pharmaceutically acceptable carriers. As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution, which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides).

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

In one embodiment, administration of the microparticles comprising the pharmaceutical compositions of this invention or another pharmaceutical agent to be administered in addition to the pharmaceutical compositions of this invention provides local or plasma concentrations sustained at approximately constant values over the intended period of release (e.g., up to 2 to 24 hours, to enable dosing once, twice, three times, four times or more than four times per day). The microparticle formulations may allow patients to take treatments less frequently, and to receive more prolonged and steadier relief.

Solid compositions of a similar type may also be employed as fillers in soft and hard-filled gelatin capsules using such excipients as lactose or milk sugar as well as high molecular weight polyethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Preferred suitable daily oral dosages for the compounds of the inventions described herein are on the order of about 1.5 mg to about 20 mg. Dosing schedules may be adjusted to provide the optimal therapeutic response. For example, administration can be one to three times daily for a time course of one day to several days, weeks, months, and even years, and may even be for the life of the patient. Practically speaking, a unit dose of any given composition of the invention or active agent can be administered in a variety of dosing schedules, depending on the judgment of the clinician, needs of the patient, and so forth. The specific dosing schedule will be known by those of ordinary skill in the art or can be determined experimentally using routine methods. Exemplary dosing schedules include, without limitation, administration five times a day, four times a day, three times a day, twice daily, once daily, every other day, three times weekly, twice weekly, once weekly, twice monthly, once monthly, and so forth. Unit dose preparations can contain a compound of formula I in the range of about 1.5 to about 20 mg. Preferably, a unit dose form can contain about 1.5 to about 10 mg of a compound of formula I, while even more preferably a unit dose can have about 1.5 to about 5 mg of a compound of formula I.

Pharmaceutical kits useful in treating the diseases associated with opioid activity or blockade comprise a therapeutically effective amount of a peripheral acting compound and the compounds of formula I of the invention, in one or more sterile containers, are also within the ambit of the present invention. Sterilization of the container may be carried out using conventional sterilization methodology well known to those skilled in the art. The sterile containers of materials may comprise separate containers, or one or more multi-part containers, as exemplified by the UNIVIAL® two-part container (available from Abbott Labs, Chicago, Ill.), as desired. The peripheral acting compound and the compound of formula I may be separate, or combined into a single dosage form as described above. Such kits may further include, if desired, one or more of various conventional pharmaceutical kit components, such as for example, one or more pharmaceutically acceptable carriers, additional vials for mixing the components, etc., as will be readily apparent to those skilled in the art. Instructions, either as inserts or as labels, indicating quantities of the components to be administered, guidelines for administration, and/or guidelines for mixing the components, may also be included in the kit.

Unless otherwise defined, all technical and scientific terms used herein are accorded the meaning commonly known to one with ordinary skill in the art. All publications, patents, published patent applications, and other references mentioned herein are hereby incorporated by reference in their entirety.

### SYNTHETIC METHODS

The compounds and processes of the present invention will be better understood in connection with the following synthetic schemes that illustrate the methods by which the compounds of the invention may be prepared, which are intended as an illustration only and not to limit the scope of the invention. Various changes and modifications to the disclosed embodiments will be apparent to those skilled in the art and such changes and modifications including, without limitation, those relating to the chemical structures, substituents, derivatives, formulations and/or methods of the invention may be made without departing from the spirit of the invention and the scope of the appended claims.

The compounds of formula I according to the present invention may be synthesized employing methods taught, for example, in U.S. Pat. No. 5,250,542, U.S. Pat. No. 5,434,171, U.S. Pat. No. 5,159,081, U.S. Pat. No. 4,176,186 U.S. Pat. No. 6,365,594, U.S. Pat. No. 6,784,187 and U.S. Pat. No. 5,270,328, the disclosures of which are hereby incorporated herein by reference in their entireties. Synthetic methodology for indolylmorphinans is described in Jones et. al. Journal of Medicinal Chemistry, 1998, 41, 4911. Synthetic methodology for pyridomorphinans is described in Ananthan et al., Bioorganic & Medicinal Chemistry Letters, 13, 2003, 529-532. The optically active and commercially available Naltrexone was employed as starting material in the synthesis of the present compounds may be prepared by the general procedure taught in U.S. Pat. No. 3,332,950, the disclosure of which is hereby incorporated herein by reference in its entireties. Compounds 1a and 1b were synthesized from their corresponding phenols using methodology described in the following references: U.S. Patent No. 6,784,187; Wentland et al. Bioorganic & Medicinal Chemistry Letters, 2001, 11, 623; Wentland et al., Bioorganic & Medicinal Chemistry Letters, 2001, 11, 1717, Wentland et al., Bioorganic & Medicinal Chemistry Letters, 2005, 15, 2107. The corresponding phenols of 1a and 1b were converted to triflate ester by treating phenol and (CF₃SO₂)₂O and pyridine in CH₂Cl₂ or PhN(SO₂CF₃)₂ and triethylamine in methylene chloride. The triflates were converted to a nitrile using Zn(CN)₂, Pd(PPh₃)₄ followed by the hydrolysis of nitrile using KOH/t-BuOH to give the carboxamide product.

### EXAMPLES

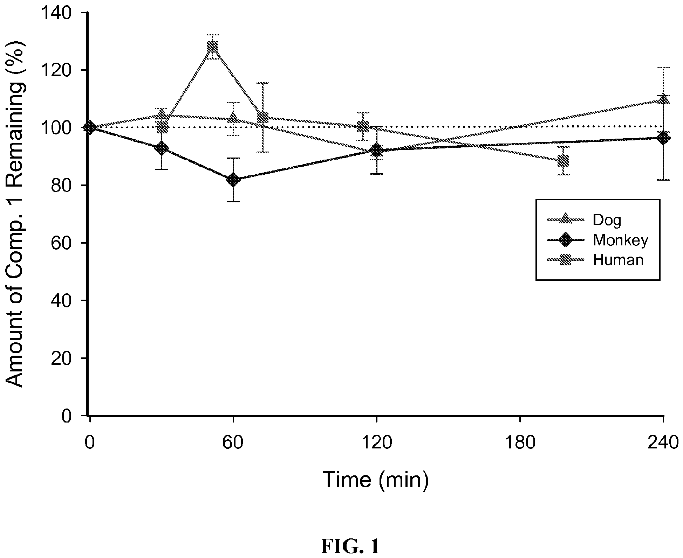

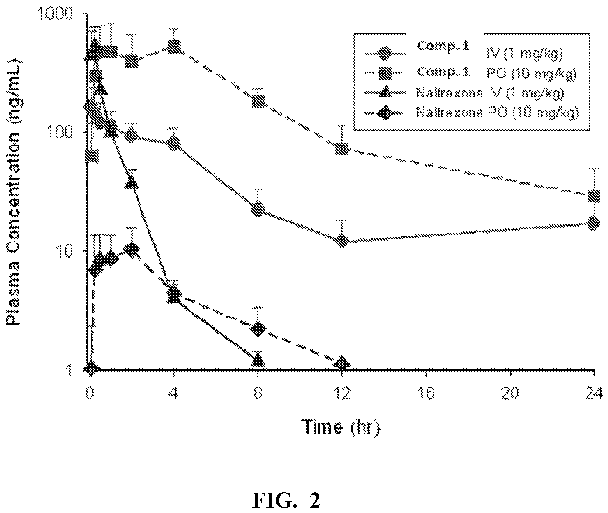

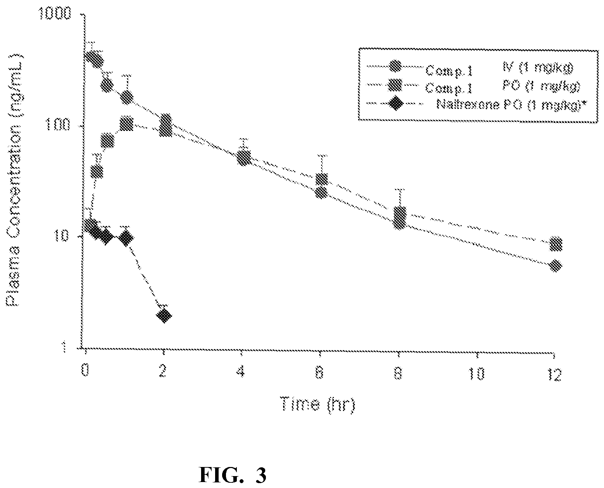

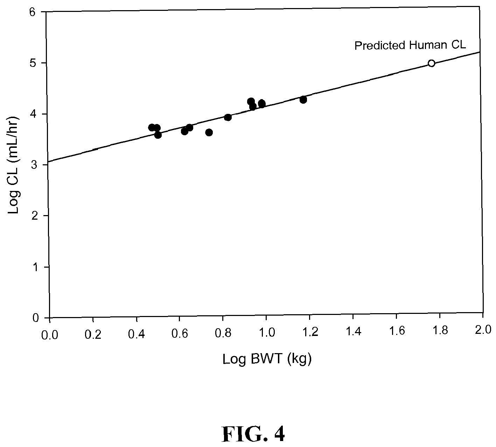

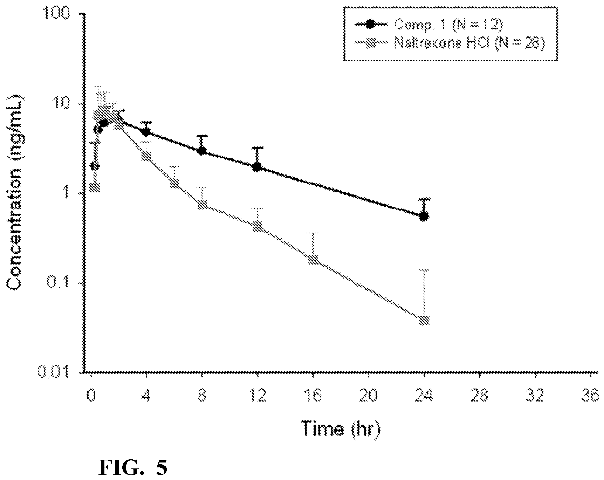

The compounds and processes of the present invention will be better understood in connection with the following examples, which are intended as an illustration only and not to limit the scope of the invention. Various changes and modifications to the disclosed embodiments will be apparent to those skilled in the art and such changes and modifications including, without limitation, those relating to the chemical structures, substituents, derivatives, formulations and/or methods of the invention may be made without departing from the spirit of the invention and the scope of the appended claims.
**Example 1:** Metabolic stability of Compound 1 in cryopreserved hepatocytes (liver cells): Compound 1 was incubated with cryopreserved hepatocytes from rat, dog, monkey, and human at concentrations of 0.5 and 5 µM. The incubations were performed in triplicate (0.5 x 10⁶ cells per incubation, 37°C, 5% CO₂, gentle shaking). The incubations were terminated at 0, 30, 60, 120 and 240 minutes. Heat treated samples were included as negative controls. After termination of incubation, Compound 1 was detected by LC-MS/MS and the loss of parent Compound 1 was determined. Figure 1 shows the metabolic stability of Compound 1 in rat, dog, monkey and human liver cells.
**Example 2:** Pharmacokinetic analysis of Compound 1: The PK of Compound 1 and the reference compound Naltrexone was determined following IV (1 mg/ kg) and PO (10 mg/kg or 1 mg/ kg) administration. Concentrations of Compound 1 and naltrexone were determined by LC-MS/MS. The PK parameters were determined by noncompartmental analysis using WiNonlin (v5.1). Figures 2-4 show the PK profiles of naltrexone and Compound 1.
Clearance of Compound-1 after IV administration to dog and monkey was utilized to predict clearance in human. Figure 5 shows the predictive human clearance as determined by allometric scaling.
**EXAMPLE 3:** The pharmacokinetic profile of naltrexone was compared to compound 1 by oral administration to humans. A single oral dose of Naltrexone.HCl (50mg) was administered. In case of Compound 1, a single oral dose of 5 mg was administered. The results are shown in Figure 5.

The patent and scientific literature referred to herein establishes the knowledge that is available to those with skill in the art. All United States patents and published or unpublished United States patent applications cited herein are incorporated by reference. All published foreign patents and patent applications cited herein are hereby incorporated by reference. All other published references, documents, manuscripts and scientific literature cited herein are hereby incorporated by reference.

While this invention has been particularly shown and described with references to preferred embodiments thereof, it will be understood by those skilled in the art that various changes in form and details may be made therein without departing from the scope of the invention encompassed by the appended claims.

Aspects and features of the present disclosure are set out in the following numbered clauses which contain the subject matter of the claims of the parent application as filed.
1. A method of treating a disease associated with opioid receptor activity or blockade by the oral administration of a compound of formula I: 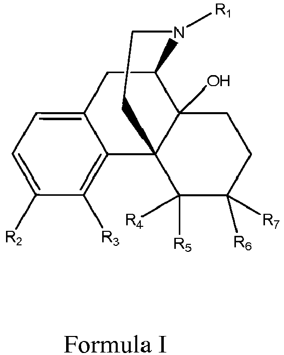 or a pharmaceutically acceptable salt, ester or prodrug thereof having high oral bioavailability, wherein;
   R₁ is -(CH₂)ₙ-c-C₃H₅, -(CH₂)ₙ-c-C₄H₇, -(CH₂)ₙ-c-C₅H₉, -(CH₂)ₙ-CH=CH₂ or - (CH₂)ₙ-CH=C(CH₃)₂ wherein n is independently 0, 1, 2 or 3;
   R₂ is -CONH₂ or -CSNH₂;
   R₃ and R₄ are independently H, -OH or together R₃ and R₄ form an -O- or -S-group;
   R₅ is H or C₁₋C₈ alkyl; and
   R₆ and R₇ are independently H, -OH, OCH₃ or together R₆ and R₇ form a =O or =CH₂ group;
   wherein, said compound of formula I is administered in a daily dosage of about 3 to about 30 mg/ day.
2. A method according to clause 1, wherein said compound of formula is: 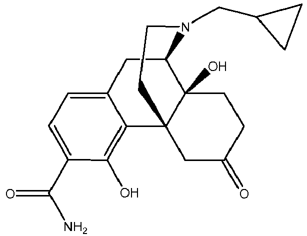
3. A method according to any of clauses 1-2, wherein said disease is alcohol addiction.
4. A method according to any one of clauses 1-3, wherein said compound of formula I is administered in a daily dose of about 3 to about 15 mg/day.
5. A method according to clause 4, wherein said daily dose is about 5 mg/day.

## Claims

1. A compound of formula: 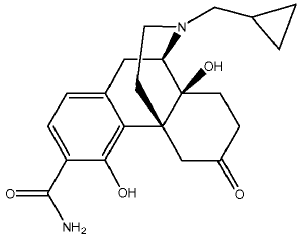 or a pharmaceutically acceptable salt or ester thereof for use in treating a disease associated with opioid receptor activity or blockade by the oral administration of said compound, wherein, said compound is administered in a daily dosage of 1.5 to 20 mg/ day.

2. The compound for use according to claim 1, wherein said compound is administered in a daily dose of 1.5 to 5 mg/day.

3. A compound of formula: 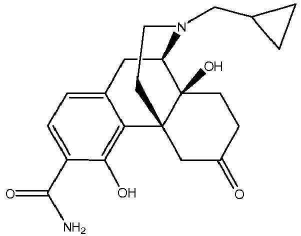 or a pharmaceutically acceptable salt or ester thereof for use in treating a disease associated with opioid receptor activity or blockade by oral administration to a subject in need thereof wherein said compound is administered in a unit dose of about 1.5 to about 5 mg.

4. The compound for use according to claim 3, wherein administration is five times a day, four times a day, three times a day, twice daily, once daily, every other day, three times weekly, twice weekly, once weekly, twice monthly, or once monthly.

5. The compound for use according to claim 4, wherein administration is once daily.

6. The compound for use according to any one of claims 1-5, wherein the disease is selected from drug addiction, opioid addiction, alcohol addiction, drug overdose, mental illness and depression.

7. The compound for use according to claim 6, wherein said disease is depression.

8. The use of a compound of formula: 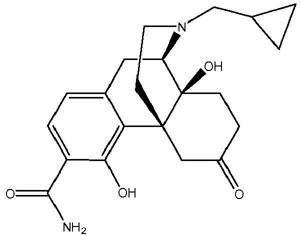 or a pharmaceutically acceptable salt or ester thereof in the manufacture of a medicament for oral administration of said compound, for the treatment of a disease associated with opioid receptor activity or blockade, wherein said compound is administered in a daily dosage of 1.5 to 20 mg/ day.

9. The use according to claim 8, wherein said compound is administered in a daily dose of 1.5 to 5 mg/day.

10. The use of a compound of formula: 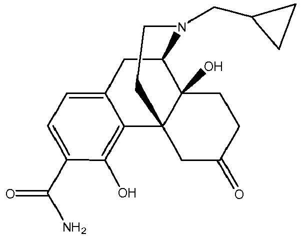 or a pharmaceutically acceptable salt or ester thereof in the manufacture of a medicament for oral administration of said compound, for the treatment of a disease associated with opioid receptor activity or blockade, wherein said compound is administered in a unit dose of about 1.5 to about 5 mg.

11. The compound for use according to claim 10, wherein administration is five times a day, four times a day, three times a day, twice daily, once daily, every other day, three times weekly, twice weekly, once weekly, twice monthly, or once monthly.

12. The compound for use according to claim 11, wherein administration is once daily.

13. The use according to any of claims 8-12, wherein the disease is selected from drug addiction, opioid addiction, alcohol addiction, drug overdose, mental illness and depression.

14. A compound for use according to claim 13, wherein said disease is depression.
